(19) Europäisches Patentamt
European Patent Office
Office européen des brevets

(11) **EP 3 085 335 A1**

(12) **EUROPEAN PATENT APPLICATION**
published in accordance with Art. 153(4) EPC

(43) Date of publication:
**26.10.2016 Bulletin 2016/43**

(51) Int Cl.:
***A61F 2/06*** *(2006.01)*     ***A61L 27/00*** *(2006.01)*

(21) Application number: **14871178.1**

(86) International application number:
**PCT/JP2014/083266**

(22) Date of filing: **16.12.2014**

(87) International publication number:
**WO 2015/093480 (25.06.2015 Gazette 2015/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.12.2013   JP 2013261755**

(71) Applicant: **Toray Industries, Inc.**
**Tokyo 103-8666 (JP)**

(72) Inventors:
• **TSUCHIKURA, Hiroshi**
  **Otsu-shi**
  **Shiga 520-2141 (JP)**
• **YAMADA, Satoshi**
  **Otsu-shi**
  **Shiga 520-2141 (JP)**

• **KUWABARA, Atsushi**
  **Otsu-shi**
  **Shiga 520-2141 (JP)**
• **TOKUDA, Akihiro**
  **Otsu-shi**
  **Shiga 520-2141 (JP)**
• **TANAHASHI, Kazuhiro**
  **Otsu-shi**
  **Shiga 520-8558 (JP)**
• **FUJITA, Masaki**
  **Otsu-shi**
  **Shiga 520-8558 (JP)**
• **KADOWAKI, Koji**
  **Otsu-shi**
  **Shiga 520-8558 (JP)**

(74) Representative: **Kador & Partner**
**Corneliusstraße 15**
**80469 München (DE)**

(54) **ARTIFICIAL BLOOD VESSEL**

(57)     Provided is a textile vascular prosthesis with reduced leakage of blood through the voids between the fibers. In particular, provided is a vascular prosthesis with multi-layer tubular woven structure, the prosthesis comprising two types of yarns, the yarns being a microfiber multifilament yarn with a monofilament fineness of 0.5 dtex or less as a warp yarn for mainly forming an inner layer to be in contact with a blood flow and a multifilament yarn with a monofilament fineness of 1.0 dtex or more as a warp yarn for mainly forming an outer layer, the inner layer having an apparent cover factor of 2000 or more.

Fig. 1

**Description**

TECHNICAL FIELD

The present invention relates to a vascular prosthesis with a low permeability of blood. In particular, the present invention relates to a textile vascular prosthesis with reduced leakage of blood through the voids between the fibers.

BACKGROUND ART

Textile vascular prostheses with substantially no leakage of blood are conventionally designed to have a structure that does not allow blood to infiltrate into the textile to prevent the leakage of blood. However, in order to produce such a blood-impermeable structure, fibers are needed to be densely woven. A densely woven fabric tends to be stiff due to the closely packed fibers. A vascular prosthesis made of such a stiff fabric is often too stiff to be surgically sewn into a calcified artery that results from arteriosclerosis or a thinned arterial wall at a risk of tearing due to an aneurysm.

Under such a limitation, the weaving or knitting designs for vascular prostheses have been variously modified to develop vascular prostheses with as much flexibility as possible and with reduced leakage of blood.

Textile vascular prostheses designed to have a fiber structure with reduced leakage of blood can be classified based on their basic structure into those with a knitted structure and those with a woven structure. Knitted vascular prostheses are produced by a simple production process and have flexibility, but have poor shape-retaining properties and often have porous structure, as a result of which the leakage of blood through the voids between the fibers tends to occur. For this reason, knitted vascular prostheses are used for repair of arteries in the peripheral extremities etc. , for which the leakage of blood is not immediately life-threatening.

A woven structure can be designed to have smaller voids between the fibers than those in a knitted structure, and thus can more efficiently prevent the leakage of blood. Woven vascular prostheses are therefore used in aortic surgery. Another fabric structure besides knitted and woven structures is a nonwoven structure. Nonwovens have an uneven structure and poor shape-retaining properties, and hence are not used for vascular prostheses.

Blood pressure is maintained at a certain high level in a living body, and due to this, the leakage of blood through the voids between the fibers is difficult to be avoided. Accordingly, before use of a textile vascular prosthesis in vascular surgery, the so-called preclotting is usually performed. Preclotting is a pre-implantation procedure in which a vascular prosthesis is brought into contact with blood for artificial formation of thrombi and temporal clogging of the voids between the fibers with the thrombi.

In today' s vascular surgery, however, heparin is often used to prevent the coagulation of the blood. Consequently, it is often the case that clogging by preclotting becomes insufficient, which leads to a risk that the leakage of blood may occur and may result in massive bleeding after surgery. Another risk is that, after surgery, fibrin produced by preclotting may begin to be dissolved by fibrinolysis as a natural phenomenon, and then the thrombus tissue artificially produced in the voids between the fibers may be easily broken, which may be another cause of life-threatening massive bleeding after surgery.

For these reasons, in cases where such a medical material is used in aortic and cardiac surgery using a large amount of heparin, in particular when the medical material is a fabric that causes the leakage of a certain amount of blood, a biodegradable substance such as collagen and gelatin is applied to the medical material to prevent the leakage of blood by not allowing the permeation of the blood into the medical material. This technique is utilized for the so-called coated vascular prosthesis and the so-called coated patch, and they are already commercially available (Non Patent Literature 1 and 2).

Patent Literature 1 discloses an invention of a medical material made of fibers, comprising a nonwoven fabric formed from microfibers of 0.5 denier or less. Patent Literature 2 discloses an invention of a multi-layer woven fabric made from microfibers of 0.5 dtex or less and fibers of 1 dtex or more.

CITATION LIST

PATENT LITERATURE

Patent Literature 1: JP 11-164881 A
Patent Literature 2: JP 2005-124959 A

NON PATENT LITERATURE

Non Patent Literature 1: Scott SM, Gaddy LR, Sahmel R, Hoffman H. A collagen coated vascular prosthesis. J Cardiovasc Surg. 1978; 28; 498-504.
Non Patent Literature 2: Noishiki Y. Chvapil M. Healing pattern of collagen-impregnated and preclotted vascular grafts in dogs. Vasc Surg. 1987; 21; 401-411.

SUMMARY OF INVENTION

TECHNICAL PROBLEM

In the techniques in Non Patent Literature 1 and 2, many of the substances (such as collagen and gelatin) used to create clogging on the surface of a coated vascular prosthesis or a coated prosthetic patch are naturally occurring substances, and hence the stabilization of the quality of the substances is very difficult. Therefore these substances are not suitable for industrial application.

In Patent Literature 1, the nonwoven fabric contains the microfibers at a low density and thus has many voids. Therefore, unless the nonwoven fabric is made into a double-layer composite with a woven fabric, a desired level of reduced leakage of blood and stable performance cannot be achieved. In Patent Literature 2, the woven fabric is produced to have a low density so as to achieve flexibility, and the leakage of blood is intended to be prevented by thrombus formation on the surface of the microfibers, which leads to clogging of the voids in the woven fabric. Due to this configuration, its performance on the prevention of the leakage of blood varies and is not stable.

The present invention was made to solve the above problems associated with conventional techniques. An object of the present invention is therefore to provide a textile vascular prosthesis with reduced leakage of blood through the voids between the fibers.

SOLUTION TO PROBLEM

Conventionally, a vascular prosthesis that contains a multifilament yarn with a high monofilament fineness (high thickness) in part of the warp to prevent the deterioration of strength due to hydrolysis is known. A vascular prosthesis that contains a microfiber multifilament yarn to promote rapid adherence and growth of vascular endothelial cells is also known. However, a vascular prosthesis that effectively prevents the leakage of blood has not been proposed.

The inventors conducted extensive research to solve the above problems and completed the present invention.

The inventors found that it is important to increase the weave density of the inner wall surface mainly formed of a microfiber multifilament yarn to prevent the leakage of blood. The present invention was made based on this finding and includes the following.

(1) A vascular prosthesis with multi-layer tubular woven structure, the prosthesis comprising two types of yarns, the yarns being a microfiber multifilament yarn with a monofilament fineness of 0.5 dtex or less as a warp yarn for mainly forming an inner layer to be in contact with a blood flow and a multifilament yarn with a monofilament fineness of 1.0 dtex or more as a warp yarn for mainly forming an outer layer, the inner layer having an apparent cover factor of 2000 or more.

(2) The vascular prosthesis of the above (1), wherein the degree of exposure of the multifilament yarn on the inner surface is 20% or less.

(3) The vascular prosthesis of the above (1), wherein at least part of the weft of the inner layer comprises a microfiber multifilament yarn with a monofilament fineness of 0.5 dtex or less.

(4) The vascular prosthesis of the above (1), wherein at least part of the weft comprises a monofilament yarn with a monofilament fineness of 15 dtex or more.

ADVANTAGEOUS EFFECTS OF INVENTION

The vascular prosthesis of the present invention with the above structure has various properties required of it and reduced leakage of blood through the voids between the fibers.

BRIEF DESCRIPTION OF DRAWINGS

Fig. 1 is a magnified photograph of the inner surface of a vascular prosthesis cut open in the longitudinal direction, for the determination of an apparent cover factor of the inner layer (magnification: 150x).

Fig. 2 is a close-up schematic view of a principal part of Fig. 1, for describing the determination of an apparent cover factor of the inner layer.

DESCRIPTION OF EMBODIMENTS

Vascular prosthesis comprising two types of yarns, i.e., a microfiber multifilament yarn with a monofilament fineness of 0.5 dtex or less as a warp yarn for mainly forming the inner layer and a multifilament yarn with a monofilament fineness of 1.0 dtex or more as a warp yarn for mainly forming the outer layer, the inner layer having an apparent cover factor of 2000 or more

In order to prevent the leakage of blood, fibers are needed to be densely woven, but a densely woven fabric tends to be stiff. The inner layer formed of a stiff fabric may cause kinking and have an uneven surface, which may result in the restriction of the blood flow. The vascular prosthesis of which the warp of the inner layer comprises a microfiber multifilament yarn with a monofilament fineness of 0.5 dtex or less has a dense but flexible structure. The vascular prosthesis of which the warp of the outer layer comprises a multifilament yarn with a monofilament fineness of 1.0 dtex or more has high mechanical strength. In the case of long-term use of the implant, the deterioration of the strength due to hydrolysis is concerned, depending on the type of the polymer used as the material of the fibers, and therefore the warp of the outer layer preferably comprises a multifilament yarn with a monofilament fineness of 2.0 dtex or more. However, when the monofilament fineness of the multifilament yarn is too large, the vascular prosthesis is too stiff to bend, and may cause kinking and may cause exposure of the multifilament yarn on the inner surface. Therefore the multifilament yarn contained in the warp of the outer layer is preferably 10.0 dtex or less. An apparent cover factor of the inner layer indicates the degree of the presence of voids between the fibers (packing density) in the inner layer. A smaller apparent cover factor means a larger amount of voids between the fibers. The inner layer with an apparent cover factor of 2000 or more, preferably 2200 or more, has a structure in which the microfiber multifilament yarn is densely packed and effectively prevents the leakage of blood. In addition, the densely packed microfiber multifilament yarn enhances the adherence and growth of vascular endothelial cells and promotes the settlement of the adherent vascular endothelial cells. The inner layer preferably has a high apparent cover factor for the settlement of adherent vascular endothelial cells, but a too high cover factor will deteriorate the flexibility of the vascular prosthesis and reduce the weaving efficiency during the production of the vascular prosthesis. The maximum value of the cover factor will vary depending on the stiffness of the fibers to be used, the performance of the loom to be used, and the weave pattern to be used, but the apparent cover factor of the inner layer is preferably 3000 or less.

Vascular prosthesis in which the degree of exposure of the multifilament yarn on the inner surface is 20% or less

The exposure of the multifilament yarn on the inner surface will reduce the effect of promoting the growth of vascular endothelial cells, compared with the cases where the microfiber multifilament yarn is exposed on the inner surface. In addition, the exposed multifilament yarn often creates a space between itself and the adjacent multifilament yarn, and the space tends to cause the leakage of blood. The space also serves as a starting point of thrombus formation. Therefore the exposure of the multifilament yarn on the inner surface is not preferred. For these reasons, the degree of exposure of the multifilament yarn on the inner surface is preferably 20% or less, more preferably 5% or less, and further more preferably 1% or less.

Vascular prosthesis in which part of the weft of the inner layer comprises a microfiber multifilament yarn with a monofilament fineness of 0.5 dtex or less

The inner layer comprising a microfiber multifilament yarn with a monofilament fineness of 0.50 dtex or less in each of the warp and weft has a smaller amount of voids between the fibers, which results in further reduced leakage of blood. The inner layer comprising a microfiber multifilament yarn with a monofilament fineness of 0.4 dtex or less provides a very large number of scaffolds suitable for the adherence of vascular endothelial cells. As a result, vascular endothelial cells are well settled on the structural fibers of the inner layer of the vascular prosthesis, and vascular endothelial cells well adhere to the inner layer of the vascular prosthesis. In addition, since the microfiber multifilament yarn with a monofilament fineness of 0.5 dtex or less is contained in both of the warp and weft, the adherent vascular endothelial cells grow and freely spread over the fiber surface of the warp and weft of the inner layer of the vascular prosthesis, thereby forming a thin layer of vascular endothelial cells inside the vascular prosthesis. In order to reduce the voids between the fibers in the inner layer and to thereby reduce the leakage of blood, the monofilament fineness of the warp and weft yarns forming the inner layer is preferably 0.5 dtex or less. In order to promote the adherence of vascular endothelial cells, the monofilament fineness of the warp and weft yarns forming the inner layer is preferably 0.4 dtex or less, more preferably 0.3 dtex or less, and further more preferably 0.25 dtex or less. Inversely, when the monofilament fineness is 0.008 dtex or less, the adherence of the cells tends to be inhibited. Therefore the monofilament fineness of the warp and weft yarns forming the inner layer is preferably more than 0.008 dtex, and more preferably 0.02 dtex or more. The monofilament fineness of the warp and weft yarns forming the inner layer is further preferably 0.02 to 0.25 dtex, and particularly preferably 0.05 to 0.25 dtex.

Vascular prosthesis in which part of the weft comprises a monofilament yarn with a monofilament fineness of 15 dtex or more

The vascular prosthesis in which part of the weft comprises a monofilament yarn with a monofilament fineness of 15 dtex or more has a higher shape-retaining properties and a higher elasticity and further resists kinking (higher kink resistance). However, if this monofilament yarn is too thick, the yarn is difficult to be made into a woven fabric due to its stiffness. The thickness of the monofilament may be selected depending on the type and performance of the loom, but typically the thickness is preferably 1000 dtex or less. Such a monofilament yarn is preferably used to form the outer layer to increase the kink resistance.

As the microfiber multifilament yarn, the so-called direct spun yarn may be directly used, and a splittable yarn may be used. The splittable yarn may be the one that can be made into ultra-fine fibers by chemical or physical means. The ultra-fining process may be performed after the tubular woven fabric is formed. The ultra-fining process by chemical or physical means may be done by, for example, removing one of the components in composite fibers or splitting composite fibers into their respective component, thereby giving fibrils or ultra-fine fibers, as described in U.S. Pat. No. 3531368 and U.S. Pat. No. 3350488. By such a process, fibers with a common thickness at the time of the formation of a multi-layer tubular woven fabric can be made into ultra-fine fibers at a later process. Consequently, troubles that may occur during various processing, for example, breakage of a yarn and formation of lint during the weaving process or during various yarn processing before weaving, are minimized.

The vascular prosthesis of the present invention is preferably a double-layer woven vascular prosthesis formed by weaving two layers together by well-known technique, such as binding of the inner layer with the warp, binding of the inner layer with the weft, and binding with the multiple wefts.

Various types of organic fibers may be used as the fibers forming the vascular prosthesis of the present invention, but preferred in terms of the water absorptivity and the degradation resistance are polyester fibers. Examples of the polyester fibers include polyethylene terephthalate fibers, polybutylene terephthalate fibers, etc. The polyester fibers may be copolymerized polyester fibers produced by copolymerizing polyethylene terephthalate or polybutylene terephthalate with an acid component, for example, isophthalic acid, sodium 5-sulfoisophthalate, or an aliphatic dicarboxylic acid such as adipic acid. The fibers contained in the multifilament yarn may be a single type or an appropriate combination of different types of fibers.

The loom to be used may be a water-jet loom, an air-jet loom, a rapier loom, a shuttle loom, etc. Of these, preferred is a shuttle loom, which is excellent in weaving a tubular fabric and can give a uniform tubular structure. The weave pattern of the double-layer woven vascular prosthesis may be plain weave, twill weave or sateen weave, or modified weave thereof, or multi-layer weave. The basic weaving process for producing the vascular prosthesis of the present invention may be a known process.

The vascular prosthesis of the present invention can be used for applications involving loading of an antithrombotic agent on a vascular prosthesis. The antithrombotic agent loaded on the vascular prosthesis may be, for example, an organism-derived anticoagulant, such as heparin, low-molecular-weight heparin, urokinase, and hirudin; a synthetic anticoagulant and a synthetic antiplatelet, such as argatroban, warfarin, acetylsalicylic acid, and ticlopidine; etc. The vascular prosthesis may be loaded with a hydrophilic polymer, such as polyethylene glycol, polyvinyl alcohol, and polyvinylpyrrolidone. The loading may be performed by any method, and may be done by, for example, coating the surface of the multifilament yarn with a solution containing the above drug or polymer; or fixing the drug or polymer on the surface of the multifilament yarn through chemical reaction, such as condensation reaction, using a reactive functional group chemically introduced into the drug or polymer; or fixing the drug or polymer by radical reaction using a high energy beam; or filling the voids in the multifilament yarn with the drug or polymer through impregnation of the yarn with collagen, gelatin or hydrogel containing the drug or the polymer; or other methods. The loading of an ionic compound, such as heparin, may be done by, for example, coating the surface of the multifilament yarn with a salt of the ionic compound formed with a counterion, or binding the counterion of the ionic compound to the surface of the multifilament yarn and then binding the ionic compound to the counterion by ionic interaction. In terms of imparting high antithrombotic activity and stably maintaining the antithrombotic activity for a long period of time, preferred are fixing of the drug or polymer on the surface through chemical reaction using a reactive functional group chemically introduced into the drug or polymer, and binding of the counterion of the drug or polymer to the surface followed by ionic binding of the drug or polymer to the counterion. The loading of the drug or polymer on the multifilament yarn, as described above, for imparting antithrombotic activity may be performed before the formation of the tubular woven fabric. However, antithrombotic activity is preferably imparted after the formation of a composite tubular woven fabric in view of reduction in the production cost.

Needless to say, the vascular prosthesis of the present invention can be used for applications involving preclotting.

EXAMPLES

The present invention will be specifically described with reference to Examples, but the present invention is not limited thereto. Various alterations and modifications are possible within the technical scope of the present invention. The various types of the properties evaluated in the Examples were measured as follows.

Measurement methods

(1) Monofilament fineness

The total fineness of a yarn was determined as a mass-corrected fineness in accordance with method A in JIS L 101 (2010) 8.3.1, by setting the predetermined load at 0.045 cN/dtex. The determined total fineness was divided by the number of monofilaments to give a monofilament fineness.

(2) Apparent cover factor of inner layer

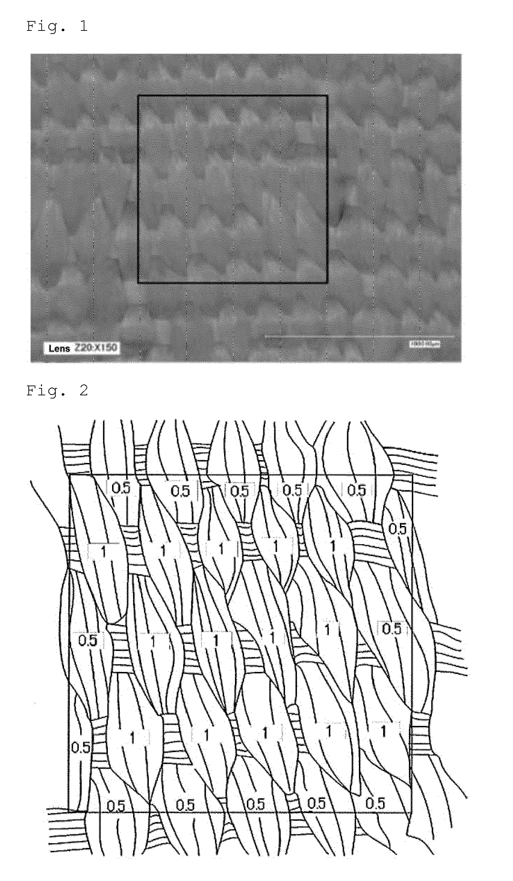

A vascular prosthesis is cut open in the longitudinal direction, and the inner surface is photographed with magnification. Fig. 1 is a magnified photograph (at a magnification of 150). Fig. 2 is a close-up schematic view of a principal part of Fig. 1, for describing the determination of an apparent cover factor of the inner layer. As shown in Fig. 2, lines parallel to the warp and weft yarns are drawn to form a square frame for defining a unit area (1 mm x 1 mm) (see the frame formed by narrow lines in Fig. 1). The number of the ridges of the warp threads of the woven structure enclosed in the square frame is counted. A ridge partially located outside the frame is counted as 0.5.

The number of the ridges in the square frame in Fig. 1 determined by counting as described above is 21 (unit number c). From the unit number c, the number of the ridges in 25.4 mm$^2$ (C) is calculated. The total fineness of the microfiber multifilament yarn is represented by Dm. Based on C and Dm, the apparent cover factor CFd of the inner surface is determined as follows:

$$CFd = [2C]^{1/2} \times 2 \times [Dm]^{1/2}.$$

In Fig. 1, Dm is 56 and CFd is 2464.

(3) Degree of exposure of multifilament yarn on inner surface

In the determination of the apparent cover factor CFd of the inner surface, 100 ridges of the warp yarn are arbitrarily selected. Within the selected ridges, the number of the ridges of the multifilament yarn containing a monofilament with a large fineness (1.0 dtex or more) is counted. The percentage of the number of the ridges (Ma) is taken as the degree of exposure on the inner surface (%).

(4) Leakage of blood

One side of a vascular prosthesis was closed and the other side was connected with a tube or other devices for feeding bovine blood at 25°C. The bovine blood was fed to the vascular prosthesis for 20 minutes, until the whole vascular prosthesis was fully impregnated with the blood, under the conditions that the pressure applied to the inside of the vascular prosthesis was 16 kPa, so that the blood permeated from the inside to the outside of the prosthesis. After that, the blood that permeated through the vascular prosthesis was collected for 5 minutes. The amount of the blood (mL) was divided by the inner surface area (cm$^2$) of the vascular prosthesis and unit time (min). The obtained value was taken as the amount of the leakage of the blood at 16 kPa.

Example 1

A polyethylene terephthalate microfiber multifilament yarn of 144 filaments with a monofilament fineness of 0.23 dtex and a total fineness of 33 dtex and a polyethylene terephthalate multifilament yarn of 24 filaments with a monofilament fineness of 2.33 dtex and a total fineness of 56 dtex were prepared as warp yarns. Sizing of each yarn was performed with a single-end sizing machine. In order to form a warp of 25 mm in width, 600 warp ends were arranged so that two ends of the microfiber multifilament yarn alternated with one end of the multifilament yarn. The warp was threaded on a narrow-width double-shuttle dobby loom.

A polyethylene terephthalate multifilament yarn of 72 filaments with a monofilament fineness of 0.46 dtex and a total fineness of 33 dtex was prepared as a weft yarn and used to weave the weave pattern shown in Table 1 (weave pattern 1) at 750 picks per 25.4 mm.

Table 1

| Weave pattern 1 | | | | | | | | | | | | | | |
|---|---|---|---|---|---|---|---|---|---|---|---|---|---|---|
| Type of weft yarn | Shuttle | Picks | | | | | | | | | | | | |
| 33-72 | Back | 8 | | | | | | 6 | | | | | | |
| 33-72 | Front | 7 | | 2 | | | | 6 | | 8 | | | | |
| 33-72 | Back | 6 | 1 | 2 | 3 | 4 | 5 | 6 | 7 | 8 | | 10 | 11 | 12 |
| 33-72 | Front | 5 | 1 | 2 | 3 | | 5 | 6 | 7 | 8 | | | 11 | 12 |
| 33-72 | Back | 4 | | | | | | | | | | | | 12 |
| 33-72 | Front | 3 | | | | | 5 | | | | | | 11 | 12 |
| 33-72 | Back | 2 | 1 | 2 | | 4 | 5 | 6 | 7 | 8 | 9 | 10 | 11 | 12 |
| 33-72 | Front | 1 | | 2 | | 4 | 5 | 6 | | 8 | 9 | 10 | 11 | 12 |
| Heddle | | | 1 | 2 | 3 | 4 | 5 | 6 | 7 | 8 | 9 | 10 | 11 | 12 |
| Type of warp yarn | | | 33 dtex, 144 f | 33 dtex, 144 f | 55 dtex, 24 f | 33 dtex, 144 f | 33 dtex, 144 f | 55 dtex, 24 f | 33 dtex, 144 f | 33 dtex, 144 f | 55 dtex, 24 f | 33 dtex, 144 f | 33 dtex, 144 f | 55 dtex, 24 f |

The greige fabric produced as above was scoured. Into the obtained vascular prosthesis, a stainless steel stick of 15.5 mm in outer diameter was inserted and the prosthesis was heat-set at 170°C. The thus produced vascular prosthesis was subjected to the evaluation of the apparent cover factor of the inner layer, the degree of exposure on the inner surface, and the leakage of blood. The results are shown in Table 3. The leakage of blood was sufficiently low and suitable for practical use.

Example 2

A vascular prosthesis was produced in the same manner as in Example 1 except that the weave pattern shown in Table 2 (weave pattern 2) was woven at 1450 picks per 25.4 mm. The thus produced vascular prosthesis was subjected to the evaluation of the apparent cover factor of the inner layer, the degree of exposure on the inner surface, and the leakage of blood. The results are shown in Table 3. The degree of exposure on the inner surface was zero, and the leakage of blood was further lower than that in Example 1.

Table 2

| Weave pattern 2 | | | | | | | | | | | | | | |
|---|---|---|---|---|---|---|---|---|---|---|---|---|---|---|
| Type of weft yarn | Shuttle | Picks | | | | | | | | | | | | |
| 33-72 | Back | 16 | | | | | | | | | | | | 12 |
| 33-72 | Front | 15 | | 2 | | | | | | | | | | 12 |
| 33-72 | Back | 14 | 1 | 2 | 3 | 4 | 5 | 6 | 7 | 8 | | 10 | 11 | 12 |
| 33-72 | Front | 13 | 1 | 2 | 3 | 4 | 5 | 6 | 7 | 8 | | | 11 | 12 |
| 33-72 | Back | 12 | | | | | | 6 | | | | | | |
| 33-72 | Front | 11 | | | | | | 6 | | 8 | | | | |
| 33-72 | Back | 10 | 1 | 2 | | 4 | 5 | 6 | 7 | 8 | 9 | 10 | 11 | 12 |
| 33-72 | Front | 9 | 1 | 2 | | | 5 | 6 | 7 | 8 | 9 | 10 | 11 | 12 |
| 33-72 | Back | 8 | | | | | | 6 | | | | | | |
| 33-72 | Front | 7 | | | | | 5 | 6 | | | | | | |
| 33-72 | Back | 6 | 1 | 2 | 3 | 4 | 5 | 6 | 7 | 8 | | 10 | 11 | 12 |
| 33-72 | Front | 5 | 1 | 2 | 3 | 4 | 5 | 6 | | 8 | | 10 | 11 | 12 |
| 33-72 | Back | 4 | | | | | | | | | | | | 12 |
| 33-72 | Front | 3 | | | | | | | | | | | 11 | 12 |
| 33-72 | Back | 2 | 1 | 2 | | 4 | 5 | 6 | 7 | 8 | 9 | 10 | 11 | 12 |
| 33-72 | Front | 1 | | 2 | | 4 | 5 | 6 | 7 | 8 | 9 | 10 | 11 | 12 |
| Heddle | | | 1 | 2 | 3 | 4 | 5 | 6 | 7 | 8 | 9 | 10 | 11 | 12 |
| Type of warp yarn | | | 33 dtex, 144 f | 33 dtex, 144 f | 55 dtex, 24 f | 33 dtex, 144 f | 33 dtex, 144 f | 55 dtex, 24 f | 33 dtex, 144 f | 33 dtex, 144 f | 55 dtex, 24 f | 33 dtex, 144 f | 33 dtex, 144 f | 55 dtex, 24 f |

Example 3

A vascular prosthesis was produced in the same manner as in Example 2 (weave pattern 2) except that the weft yarn was a polyethylene terephthalate microfiber multifilament yarn of 144 filaments with a monofilament fineness of 0.23 dtex and a total fineness of 33 dtex. The thus produced vascular prosthesis was subjected to the evaluation of the apparent cover factor of the inner surface, the degree of exposure on the inner surface, and the leakage of blood. The results are shown in Table 3. The degree of exposure on the inner surface was zero, and the leakage of blood was further lower than that in Example 2.

Example 4

A polyethylene terephthalate microfiber multifilament yarn of 144 filaments with a monofilament fineness of 0.30 dtex and a total fineness of 44 dtex and a polyethylene terephthalate multifilament yarn of 24 filaments with a monofilament fineness of 2.33 dtex and a total fineness of 56 dtex were prepared as warp yarns. A polyethylene terephthalate microfiber multifilament yarn of 144 filaments with a monofilament fineness of 0.30 tex and a total fineness of 44 dtex was prepared as a weft yarn. With the use of the above yarns, the same weave pattern as in Example 2 (weave pattern 2) was woven in the same manner as in the Example except that the picks per 25.4 mm was 1250. The thus produced vascular prosthesis was subjected to the evaluation of the apparent cover factor of the inner surface, the degree of exposure on the inner surface, and the leakage of blood. The results are shown in Table 3. The degree of exposure on the inner surface was zero, and the leakage of blood was further lower than those in Examples 1 and 2.

Example 5

A polyethylene terephthalate microfiber multifilament yarn of 630 filaments with a monofilament fineness of 0.084 dtex and a total fineness of 53 dtex and a polyethylene terephthalate multifilament yarn of 24 filaments with a monofilament fineness of 2.33 dtex and a total fineness of 56 dtex were prepared as warp yarns. A polyethylene terephthalate microfiber multifilament yarn of 630 filaments with a monofilament fineness of 0.084 dtex and a total fineness of 53 dtex was prepared as a weft yarn. With the use of the above yarns, the same weave pattern as in Example 2 (weave pattern 2) was woven in the same manner as in the Example except that the picks per 25.4 mm was 1135. The thus produced vascular prosthesis was subjected to the evaluation of the apparent cover factor of the inner surface, the degree of exposure on the inner surface, and the leakage of blood. The results are shown in Table 3. The degree of exposure on the inner surface was zero, and the leakage of blood was further lower than those in Examples 1 to 4.

Example 6

A vascular prosthesis was produced by weaving the same weave pattern as in Example 4 (weave pattern 2) in the same manner as in the Example except that the weft yarn for the back one of the two shuttles was a polyethylene terephthalate monofilament yarn of a monofilament fineness of 22 dtex. The thus produced vascular prosthesis was subjected to the evaluation of the apparent cover factor of the inner surface, the degree of exposure on the inner surface, and the leakage of blood. The results are shown in Table 3. The degree of exposure on the inner surface was zero, and the leakage of blood was at the same level as in Example 4. In terms of the kink resistance, the vascular prosthesis had better bending resistance than those in Examples 1 to 5, and thus had good shape-retaining properties.

Comparative Example 1

A vascular prosthesis was produced in the same manner as in Example 3 (weave pattern 2) except that the total number of warp ends was 480 ends/25 mm, and that the picks per 25.4 mm was 1130. The thus produced vascular prosthesis was subjected to the evaluation of the apparent cover factor of the inner surface, the degree of exposure on the inner surface, and the leakage of blood. The results are shown in Table 3. The leakage of blood was high and unsuitable for practical use.

Comparative Example 2

A vascular prosthesis was produced in the same manner as in Example 1 (weave pattern 1) except that the total number of warp ends was 480 ends/25 mm, and that the picks per 25.4 mm was 600. The thus produced vascular prosthesis was subjected to the evaluation of the apparent cover factor of the inner surface, the degree of exposure on the inner surface, and the leakage of blood. The results are shown in Table 3. The leakage of blood was very high and more unsuitable for practical use than that in Comparative Example 1.

Table 3

|  | Apparent cover factor of inner layer | Degree of exposure on inner surface | Leakage of blood (amount of leakage of blood) |
|---|---|---|---|
| Example 1 | 2302 | 23% | 1.10 |
| Example 2 | 2280 | 0% | 0.70 |
| Example 3 | 2294 | 0% | 0.08 |
| Example 4 | 2310 | 0% | 0.50 |
| Example 5 | 2240 | 0% | 0.05 |
| Example 6 | 2310 | 0% | 0.50 |
| Comparative Example 1 | 1820 | 0% | 3.30 |
| Comparative Example 2 | 1793 | 25% | 6.20 |

INDUSTRIAL APPLICABILITY

The present invention is suitable as a vascular prosthesis used in various surgical operations.

**Claims**

1. A vascular prosthesis with multi-layer tubular woven structure, the prosthesis comprising two types of yarns, the yarns being a microfiber multifilament yarn with a monofilament fineness of 0.5 dtex or less as a warp yarn for mainly forming an inner layer to be in contact with a blood flow and a multifilament yarn with a monofilament fineness of 1.0 dtex or more as a warp yarn for mainly forming an outer layer, the inner layer having an apparent cover factor of 2000 or more.

2. The vascular prosthesis of claim 1, wherein the degree of exposure of the multifilament yarn on the inner surface is 20% or less.

3. The vascular prosthesis of claim 1, wherein at least part of the weft of the inner layer comprises a microfiber multifilament yarn with a monofilament fineness of 0.5 dtex or less.

4. The vascular prosthesis of claim 1, wherein at least part of the weft comprises a monofilament yarn with a monofilament fineness of 15 dtex or more.

Fig. 1

Fig. 2

## INTERNATIONAL SEARCH REPORT

| International application No. |
|---|
| PCT/JP2014/083266 |

A. CLASSIFICATION OF SUBJECT MATTER
*A61F2/06*(2013.01)i, *A61L27/00*(2006.01)i

According to International Patent Classification (IPC) or to both national classification and IPC

B. FIELDS SEARCHED

Minimum documentation searched (classification system followed by classification symbols)
A61F2/06, A61L27/00

Documentation searched other than minimum documentation to the extent that such documents are included in the fields searched
Jitsuyo Shinan Koho          1922–1996   Jitsuyo Shinan Toroku Koho   1996–2015
Kokai Jitsuyo Shinan Koho    1971–2015   Toroku Jitsuyo Shinan Koho   1994–2015

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 61-58190 B2  (Toray Industries, Inc.), 10 December 1986 (10.12.1986), entire text; all drawings (Family: none) | 1-4 |
| A | JP 2011-245282 A  (Toray Industries, Inc.), 08 December 2011 (08.12.2011), entire text; all drawings & US 2013/0041452 A1    & EP 2564810 A1 & WO 2011/136243 A1 | 1-4 |
| A | JP 2005-124959 A  (Yasuharu NOISSHIKI), 19 May 2005 (19.05.2005), entire text; all drawings (Family: none) | 1-4 |

☐ Further documents are listed in the continuation of Box C.          ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 09 March 2015 (09.03.15) | 24 March 2015 (24.03.15) |

| Name and mailing address of the ISA/ Japan Patent Office 3-4-3,Kasumigaseki,Chiyoda-ku, Tokyo 100-8915,Japan | Authorized officer |
|---|---|
|  | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11164881 A
- JP 2005124959 A
- US 3531368 A
- US 3350488 A

**Non-patent literature cited in the description**

- **SCOTT SM ; GADDY LR ; SAHMEL R ; HOFFMAN H. A.** collagen coated vascular prosthesis. *J Cardiovasc Surg.,* 1978, vol. 28, 498-504
- **NOISHIKI Y. ; CHVAPIL M.** Healing pattern of collagen-impregnated and preclotted vascular grafts in dogs. *Vasc Surg.,* 1987, vol. 21, 401-411